# EUROPEAN PATENT SPECIFICATION

(11) **EP 4 003 317 B1**
(45) Date of publication and mention of the grant of the patent: **20.09.2023**
(21) Application number: 20747478.4
(22) Date of filing: 27.07.2020
(51) Int. Cl.: A61K 31/122, A61K 31/22, A61K 31/366, A61K 31/375, A61K 31/40, A61K 31/404, A61K 31/4418, A61K 31/455, A61K 31/47, A61K 31/505, A61K 45/06, A61P 9/10

(54) **ASCORBATE IN THE PREVENTION OF STATIN INDUCED VASCULAR CALCIFICATION**
ASCORBAT ZUR VERHINDERUNG VON STATININDUZIERTER VASKULÄRER VERKALKUNG
ASCORBATE DANS LA PRÉVENTION DE LA CALCIFICATION VASCULAIRE INDUITE PAR LA STATINE

(30) Priority: 29.07.2019 US 201916524383; 07.08.2019 EP 19190436; 05.09.2019 US 201916562313
(43) Date of publication of application: 01.06.2022
(73) Proprietor: Rath, Matthias, Henderson NV 89044 (US)
(72) Inventor: IVANOV, Vadim, San Jose, California 95138 (US); NIEDZWIECKI, Aleksandra, Henderson, NV 89044 (US); RATH, Matthias, Henderson, NV 89044 (US)
(74) Representative: Féaux de Lacroix, Stefan
(86) International application number: PCT/IB2020/057063
(87) International publication number: WO 2021/019421

(56) References cited:
- EP-A1- 1 214 893
- WO-A2-03/072013
- US-A1- 2004 009 986
- US-A1- 2004 014 712
- US-A1- 2004 023 919
- US-A1- 2009 074 883
- US-A1- 2010 021 573
- ARAD YADON ET AL: "Treatment of Asymptomatic Adults With Elevated Coronary Calcium Scores With Atorvastatin, Vitamin C, and Vitamin E The St. Francis Heart Study Randomized Clinical Trial", JOURNAL OF THE AMERICAN COLLEGE OF CARDIOLOGY, vol. 46, no. 1, 2005, pages 166-172, XP029657339, ISSN: 0735-1097, DOI: 10.1016/J.JACC.2005.02.089
- ASTRID TRION ET AL: "Modulation of calcification of vascular smooth muscle cells in culture by calcium antagonists, statins, and their combination", MOLECULAR AND CELLULAR BIOCHEMISTRY, KLUWER ACADEMIC PUBLISHERS, BO, vol. 308, no. 1-2, 2 October 2007 (2007-10-02), pages 25-33, XP019578289, ISSN: 1573-4919
- NAJWA SKAFI ET AL: "Phospholipase D: A new mediator during high phosphate-induced vascular calcification associated with chronic kidney disease :", JOURNAL OF CELLULAR PHYSIOLOGY, vol. 234, no. 4, 12 September 2018 (2018-09-12), pages 4825-4839, XP055667511, US ISSN: 0021-9541, DOI: 10.1002/jcp.27281
- PANH LOÏC ET AL: "Coronary artery calcification: From crystal to plaque rupture", ARCHIVES OF CARDIOVASCULAR DISEASE, vol. 110, no. 10, 20 July 2017 (2017-07-20), pages 550-561, XP085229835, ISSN: 1875-2136, DOI: 10.1016/J.ACVD.2017.04.003

## Description

The present invention relates to a mixture comprising L-ascorbic acid or ascorbate for use in preventing or mitigating the vascular calcification induced by statins.

Statins are also known as HMG-CoA reductase inhibitors and are a class of lipid-lowering compounds.

Low-density lipoprotein (LDL) carriers of cholesterol play an important role in the development of atherosclerosis and coronary heart disease via the mechanisms described by the lipid hypothesis. Statins are effective in lowering the LDL cholesterol and so are widely used for primary prevention in people having a high risk of cardiovascular disease, as well as in secondary prevention for those who have developed cardiovascular disease.

Upon treatment with statins, patients exhibit side effects including muscle pain, increased risk of diabetes mellitus, and abnormal blood levels of liver enzymes.

In some patients, for example lovastatin leads to myopathy and asymptomatic, but marked and persistent increases in liver transaminases. The transaminase increase produced by lovastatin and other HMG-CoA reductase inhibitors is a direct consequence of the inhibition of the mevalonate synthesis. To counteract the increased transaminase levels observed in a small number of patients, US 4,929,437 teaches the adjunct administration of an effective amount of HMG-CoA reductase inhibitor and an effective amount of coenzyme Q₁₀ in order to counter-act HMG-CoA reductase inhibitor-associated liver damage.

It has furthermore been known that statins increase vascular calcifications, which are a recognized risk factor for heart disease (Ikegami Y, Inoue I, Inoue K, Shinoda Y3, lida S1, Goto S4, Nakano T5, Shimada A1, Noda M1. The annual rate of coronary artery calcification with combination therapy with a PCSK9 inhibitor and a statin is lower than that with statin monotherapy. NPJ Aging Mech Dis 2018;4:7).

In the recent analysis of 8 prospective randomized trials using serial coronary intravascular ultrasound, Puri et al. (Puri R, Nicholls SJ, Shao M, Kataoka Y, Uno K, Kapadia SR, Tuzcu EM, Nissen SE. Impact of statins on serial coronary calcification during atheroma progression and regression. J Am Coll Cardiol 2015; 65:1273-1282) concluded that independent of their plaque-regressive effects, statins promote coronary atheroma calcification.

Still there is a controversy between arterial calcification being a well-established marker and prognoses index for cardiovascular disease development, statins stimulating effects on arterial calcification and apparent beneficial effects of statin supplementation on clinical events in CVD patients. Some researchers are providing a tending plausible explanation of these conflicting evidences to be a "special" mechanism of arterial calcification under statin treatments which results in greater lesion stability defined as fewer VH-thin-cap fibroatheromas and plaque ruptures and more calcified thick-cap fibroatheromas (Kadohira T1, Mintz GS, Souza CF, Witzenbichler B, Metzger DC, Rinaldi MJ, Mazzaferri EL Jr, Duffy PL, Weisz G, Stuckey TD, Brodie BR, Crowley A, Kirtane AJ, Stone GW, Maehara A. Impact of chronic statin therapy on clinical presentation and underlying lesion morphology in patients undergoing percutaneous intervention: an ADAPT-DES IVUS substudy. Coron Artery Dis 2017; 28:218-224).

Vascular calcification is a relevant pathophysiological process that is associated with coronary atherosclerosis, and is a prognostic marker of cardiovascular morbidity and mortality.

Vascular smooth muscle cells (SMC) have an extraordinary capacity to undergo osteoblastic phenotypical differentiation. Calcification of the intimal and/or medial vascular cell layer leads to differentiation of osteoblasts whether from a smooth muscle cell, a mesenchymal cell, or vascular pericyte, characterized, among others, by increased alkaline phosphatase activity, osteocalcin production and bone matrix secretion. Biochemical mechanisms associated with the conversion of SMC into osteoblastic cells have been elaborated, however the decisive mechanisms of what triggers and/or regulates this process have remained largely elusive.

Recent studies showed that plaque calcification is a dynamic process and related to the degree of vascular inflammation. Several inflammatory factors produced during the different phases of atherosclerosis can induce the expression and activation of osteoblastic cells located within the arterial wall, which, in turn, promote deposition of calcium.

The presence of regulatory proteins along with dedifferentiated osteoblast-like cells was demonstrated to originate from vascular smooth muscle cells (VSMCs) that were designated calcifying vascular cells. These cells are implicated in the synthesis/reabsorption of bone in atherosclerotic plaques, especially around calcification. Thus, it has been proposed that bone cell function in the vascular wall is, in some aspects, similar to that in bones. However, in vitro studies provided evidence that regulation of bone synthesis in the vascular wall and in the skeleton are different. When stimulated by oxidative stress or with oxidized LDL, osteoblasts of the skeleton and CVCs (a population of vascular cells with osteoblastic characteristics) showed opposing response, a decrease and increase of bone formation, respectively.

US 2004/0023919 A1 discloses a blood lipid ameliorant composition. The pharmaceutical composition combines atorvastatin and an ascorbic acid derivative which can be sodium ascorbate, calcium ascorbate or ascorbic acid. Effects of co-administration of atorvastatin and ascorbic acid are shown in Table 8. Blood FFA levels were reduced when administering atorvastatin and ascorbic acid, see the last entry. Compositions containing atorvastatin and ascorbic acid are shown in Tables 1 to 4. In paragraph [0008] it is stated that co-administration of atorvastatin with a certain vitamin (ascorbic acid) reduces the total cholesterol levels in the blood.

US 2004/0014712 A1 discloses the combination of simvastatin and ascorbic acid. Blood lipid peroxide levels, blood FFA levels and CPK levels were reduced by the combination of simvastatin and ascorbic acid. Corresponding pharmaceutical compositions are listed in Tables 1 to 4. In paragraph [0006] it is stated that blood lipid levels are ameliorated by this combination.

US 2004/0009986 A1 discloses a combination of pravastatin and ascorbic acid derivatives. It is stated that blood triglyceride levels could be reduced by a combination of pravastatin with ascorbic acid (and additionally tocopherol or tocopherol and riboflavin butyrate). In paragraph [0007] it is stated that the drug composition reduces triglyceride levels in the blood.

WO 03/072013 A2 discloses a combination of a statin with ascorbic acid for the treatment of psoriasis.

Y. Arad et al., Journal of the American College of Cardiology, vol. 46, no. 1, 2005, 166-172, states in the conclusion on the first page that the treatment with alpha-tocopherol, vitamin C and low doses of atorvastatin did not affect the progression of coronary calcification.

A. Trion et al., Molecular and Cellular Biochemistry, vol. 308, no. 1-2, 2007, 25-33, describes in the abstract the effects of calcium antagonists and statins on VSMC calcification in vitro. The growth medium contained ascorbic acid, however, at low levels of 50 µg/ml. It is stated that combining treatments stimulated calcification to a degree similar to that observed with atorvastatin alone.

N. Skafi et al., Journal of Cellular Physiology, vol. 234, no. 4, 2018, 4825-4839, describes on page 4827 in section 2.2 that to stimulate calcification, cells were cultured with 50 µg/ml of ascorbic acid and GP.

The object underlying the present invention is to treat or prevent the vascular calcification induced in patients by administration of statins.

The object is achieved according to the present invention by one or more of the following embodiments.

### First set of embodiments:

1. A composition mixture comprising L-ascorbic acid or ascorbate, vitamin E, vitamin B1, vitamin B2, vitamin B3, vitamin B5, vitamin B6, vitamin B12, folic acid, biotin, L-carnitine and betaine for use in treating or preventing the vascular calcification induced in patients by administration of statins.
2. The composition mixture according to embodiment 1 for use in treating patients concomitantly receiving statin treatment.
3. The composition mixture for use according to embodiment 1 or 2, wherein the composition mixture contains the following amounts:
   L-ascorbic acid or ascorbate: 300 mg to 1000.2 mg,
   vitamin E: 27.5 mg to 82.5 mg,
   vitamin B1: 3.3 mg to 9.9 mg,
   vitamin B2: 3.3 mg to 9.9 mg,
   vitamin B3: 115 mg to 350.1 mg,
   vitamin B5: 16.7 mg to 50.1 mg,
   vitamin B6: 3.3 mg to 9.9 mg,
   vitam in B 12: 10 µg to 30 µg,
   folic acid: 133.3 µg to 399.9 µg,
   biotin: 33.3 µg to 99.9 µg,
   L-carnitine: 33.3 mg to 99.9 mg,
   betaine: 23.3 mg to 69.9 mg.
4. The composition mixture for use according to one of embodiments 1 to 3, wherein the ascorbate is selected from water soluble or lipid soluble ascorbates or mixtures thereof, preferably from calcium ascorbate, magnesium ascorbate, sodium ascorbate, ascorbyl phosphate, ascorbyl palmitate or mixtures thereof.
5. A pharmaceutical composition containing at least one statin and L-ascorbic acid or ascorbate, vitamin E, vitamin B1, vitamin B2, vitamin B3, vitamin B5, vitamin B6, vitamin B12, folic acid, biotin, L-carnitine and betaine in a dosage form that allows for the concomitant administering of the at least one statin and L-ascorbic acid or ascorbate and vitamin E, vitamin B1, vitamin B2, vitamin B3, vitamin B5, vitamin B6, vitamin B12, folic acid, biotin, L-carnitine and betaine to a patient.
6. The pharmaceutical composition according to embodiment 5, wherein the at least one statin and L-ascorbic acid or ascorbate, vitamin E, vitamin B1, vitamin B2, vitamin B3, vitamin B5, vitamin B6, vitamin B12, folic acid, biotin, L-carnitine and betaine are present as a physical mixture or as separate pharmaceutical compositions intended for concomitant administration to a patient.
7. The pharmaceutical composition according to embodiment 5 or 6 for use in the prevention or treatment of cardiovascular disease.
8. The pharmaceutical composition for use according to embodiment 7, wherein the cardiovascular disease is coronary artery disease, cerebrovascular disease or peripheral vascular disease.
9. The pharmaceutical composition according to one of embodiments 5 to 8, wherein the statin is selected from the group consisting of atorvastatin, cerivastatin, fluvastatin, lovastatin, mevastatin, pitavastatin, pravastatin, rosuvastatin, simvastatin, or mixtures thereof.
10. The pharmaceutical composition according to one of embodiments 5 to 9, wherein the ascorbate is selected from water soluble or lipid soluble ascorbates or mixtures thereof, preferably from calcium ascorbate, magnesium ascorbate, sodium ascorbate, ascorbyl phosphate, ascorbyl palmitate or mixtures thereof.
11. The pharmaceutical composition according to one of embodiments 5 to 10, additionally containing coenzyme Q10 in a dosage form that allows for the concomitant administering of the at least one statin, L-ascorbic acid or ascorbate, and coenzyme Q10 to a patient.
12. The pharmaceutical composition according to one of embodiments 5 to 11, comprising a daily dosage amount of L-ascorbic acid or ascorbate from 10 mg to 100 g, and of at least one statin from 5 mg to 100 mg.
13. The pharmaceutical composition according to one of embodiments 5 to 12, containing the following amounts:
   L-ascorbic acid or ascorbate: 300 mg to 1000.2 mg,
   vitamin E: 27.5 mg to 82.5 mg,
   vitamin B1: 3.3 mg to 9.9 mg,
   vitamin B2: 3.3 mg to 9.9 mg,
   vitamin B3: 115 mg to 350.1 mg,
   vitamin B5: 16.7 mg to 50.1 mg,
   vitamin B6: 3.3 mg to 9.9 mg,
   vitamin B12: 10 µg to 30 µg,
   folic acid: 133.3 µg to 399.9 µg,
   biotin: 33.3 µg to 99.9 µg,
   L-carnitine: 33.3 mg to 99.9 mg,
   betaine: 23.3 mg to 69.9 mg
   in the pharmaceutical composition.
14. The pharmaceutical composition according to embodiment 13, further containing niacin, preferably in a mixture with the statin.
15. A composition mixture 1 comprising or consisting of vitamin C, vitamin E, vitamin B1, vitamin B2, vitamin B3, vitamin B5, vitamin B6, vitamin B12, folic acid, biotin, L-carnitine and betaine containing or consisting of the following amounts:
   vitamin C: 300 mg to 1000.2 mg,
   vitamin E: 27.5 mg to 82.5 mg,
   vitamin B1: 3.3 mg to 9.9 mg,
   vitamin B2: 3.3 mg to 9.9 mg,
   vitamin B3: 115 mg to 350.1 mg,
   vitamin B5: 16.7 mg to 50.1 mg,
   vitamin B6: 3.3 mg to 9.9 mg,
   vitamin B12: 10 µg to 30 µg,
   folic acid: 133.3 µg to 399.9 µg,
   biotin: 33.3 µg to 99.9 µg,
   L-carnitine: 33.3 mg to 99.9 mg,
   betaine: 23.3 mg to 69.9 mg
   in the composition mixture.
16. The composition mixture 1 according to embodiment 15, wherein the vitamin C is in ascorbate form, wherein the ascorbate is preferably selected from water soluble or lipid soluble ascorbates, or mixtures thereof, more preferably wherein the ascorbate is selected from a calcium ascorbate, magnesium ascorbate, sodium ascorbate, ascorbyl phosphate, ascorbyl palmitate, or mixtures thereof.
17. The composition mixture 1 according to one of embodiments 15 or 16, additionally containing a coenzyme Q10.

According to the present invention, it has been found that a composition mixture comprising L-ascorbic acid or ascorbate, vitamin E, vitamin B1, vitamin B2, vitamin B3, vitamin B5, vitamin B6, vitamin B12, folic acid, biotin, L-carnitine and betaine is effective in treating or preventing the vascular calcification in a human system, especially when co-administered with statin.

Vitamin C is an essential nutrient for certain animals including humans. Clinical trials have shown a significant positive effect of vitamin C on endothelial function when taken at doses greater than 500 mg per day. Its possible influence on the treatment or prevention of cardiovascular disease has been discussed.

Vitamin C is a very powerful antioxidant and is essential for the formation of collagen and optimum extracellular matrix (ECM). It can prevent lipoprotein deposition and development of atherosclerosis by protecting the integrity and strength of the vascular wall.

Our previous studies have shown that ascorbate can inhibit excessive proliferation and migration of SMC in vitro (Ivanov V, Ivanova S, Roomi MW, Kalinovsky T, Niedzwiecki A, Rath M. Extracellular matrix-mediated control of aortic smooth muscle cell growth and migration by a combination of ascorbic acid, lysine, proline, and catechins. J Cardiovasc Pharmacol 2007; 50:541-547). Also, dietary vitamin C is essential in prevention of lipoproteins deposition in the vascular wall and atherosclerosis in genetically engineered mice mimicking human metabolism in respect their inability to produce vitamin C and expressing human lipoprotein (a) (Cha J, Niedzwiecki A, Rath M. Hypoascorbemia induces atherosclerosis and vascular deposition of lipoprotein(a) in transgenic mice. Am J Cardiovasc Dis 2015; 5:53-62). In a clinical study, a daily micronutrient supplementation, including about 4 grams of vitamin C, was able to halt the progression of coronary calcifications in patients diagnosed with early coronary artery disease (Rath M, Niedzwiecki A. (1996) Nutritional supplement program halts progression of early coronary atherosclerosis documented by ultrafast computed tomography. J Appl Nutr 1996; 48:67-78).

Thus, it is conceivable that vitamin C plays a decisive role in regulating the cellular and extracellular architecture and function inside the vascular wall. With optimum availability of ascorbate, the integrity and stability of the vascular wall would be provided, above all, by an optimum synthesis of collagen and other ECM molecules. In chronic ascorbate deficiency or beginning scurvy, the need for compensatory mechanisms may arise to add compensatory stability to a structurally impaired vascular wall - including by means of calcification.

We investigated the effects of vitamin C on vascular SMC, human dermal fibroblasts (DF) as well as on immortalized human fetal osteoblasts (FOB) and the potential of these cells to contribute to vascular calcification. Moreover, we evaluated the role of statins in connection with this regulatory process, in light of the fact that these drugs are currently taken by millions of patients in the expectation that they curb vascular calcification. Thereby, we came to the invention as disclosed herewith.

According to the present invention, vitamin C, also known as ascorbic acid or L-ascorbic acid, is employed. As an alternative, ascorbate can be employed, wherein the ascorbate, a salt of ascorbic acid with bases or acids stronger than ascorbic acid, is preferably selected from water-soluble or lipid-soluble ascorbates or mixtures thereof and is more preferably selected from the group consisting of calcium ascorbate, magnesium ascorbate, sodium ascorbate, ascorbyl phosphate, ascorbyl palmitate or mixtures thereof.

The composition mixture 1 containing or consisting of vitamins C, E, B1, B2, B3, B5, B6, B12, folic acid, biotin, L-carnitine and betaine is (preferably) administered to patients that are treated with statins.

In the context of the present invention, statins can also be described as HMG-CoA reductase inhibitors. Thus, the statins inhibit the enzyme HMG-CoA reductase which is necessary to make cholesterol. Thus, statins relate to a class of lipid-lowering medications that reduce illness and mortality in those who are at high risk of cardiovascular disease. All suitable statins can be employed in the context of the present invention. Preferably, the stain is selected from the group consisting of atorvastatin, cerivastatin, fluvastatin, lovastatin, mevastatin, pitavastatin, pravastatin, rosuvastatin, simvastatin, or mixtures thereof, or any other type or form of statin, or from a combination of the statin with niacin.

According to one aspect of the invention, the composition mixture 1 can be administered together with one or more additional micronutrients beside L-ascorbic acid or ascorbate. For example, a pharmaceutical composition comprising composition mixture 1, and one or more additional micronutrients can be provided for this purpose.

Preferably, the one or more additional micronutrients are administered together with composition mixture 1 and with statin. The one or more micronutrients are preferably selected from trace minerals, vitamins different from composition mixture comprising L-ascorbic acid or ascorbate, vitamin E, vitamin B1, vitamin B2, vitamin B3, vitamin B5, vitamin B6, vitamin B12, folic acid, biotin, L-carnitine and betaine, and mixtures thereof. Trace minerals are only required in small amounts (traces) by humans.

Trace minerals are preferably selected from boron, cobalt (preferably as a component of vitamin B12), chromium, copper, iodine, iron, manganese, molybdenum, selenium, zinc, and mixtures thereof.

Vitamins different from vitamin C are preferably selected from vitamin B complex, vitamin B1 (thiamin), vitamin B2 (riboflavin), vitamin B3 (niacin), vitamin B5 (pantothenic acid), vitamin B6 group including pyridoxine, pyridoxal-5-phosphate, and pyridoxamine, vitamin B7 (biotin), vitamin B9 (folate or folic acid), vitamin B12 (cobalamin), comprised in the composition mixture, choline, vitamin A (e.g. retinol or provitamin A carotenoids), vitamin D, including ergocalciferol and cholecalciferol, vitamin E (tocopherols and tocotrienols), vitamin K including vitamin K1 (phylloquinone) and vitamin K2 (menaquinone), carotenoids, including alpha carotene, beta carotene, cryptoxanthin, lutein, lycopene and Zeaxanthin. Micronutrients comprised in the composition mixture include folic acid, biotin, L-carnitine and betaine.

Amino acids and their derivatives include betaine and L-carnitine. Further micronutrients comprise vitamins B6 and B12, folic acid and betaine. A preferred combination of micronutrients is contained in composition mixture 1 as outlined below.

When a patient is treated with statins, the daily dosage amount can be from the lowest to the highest commercially available or clinically applicable dose. The dosage amount is preferably in the range of from 5 to 100 mg, preferred 10 to 80 mg, more preferably 10 to 40 mg, most preferably 10 to 20 mg.

The amount of L-ascorbic acid or ascorbate administered to a patient receiving statin treatment is preferably 10 mg to 100 g, more preferably 100 mg to 10 g, most preferably 200 mg to 5 g daily dosage.

It is possible to administer composition mixture 1 as described below simultaneously with the statin, for example in a tablet containing both, composition mixture 1 as described below, and statin. Furthermore, it is possible to administer composition mixture 1 as described below and statin in separate pharmaceutical compositions, but concomitantly. The term "concomitantly" means that the administration of both active ingredients takes place within a time range of from 0 to 5 hours, preferably 0 to 3 hours, more preferably 0 to 1 hours, based on one administration per day.

Since both statins and L-ascorbic acid or ascorbate (as well as other/additional micronutrients different from vitamin C) are well established for an individual and separate administration to patients in need thereof, the known pharmaceutical or nutritional compositions or mixture 1 as described below can be employed according to the present invention while ensuring the concomitant use of both active ingredients, the mixture 1 and the statin.

A micronutrient composition that contains ascorbate and can advantageously be employed in combination with statins is mixture 1. Mixture 1 contains vitamins, L-carnitine and betaine, and specifically contains or consists of vitamins C, E, B1, B2, B3, B5, B6, B12, folic acid, biotin, L-carnitine and betaine, and can be employed for daily nutritional supplementation. Typically, three tablets are taken for a day (one tablet three times a day at meal times with plenty of liquid (water, juice, tea)).

Composition mixture 1 contains each nutrient in the range for one to three tablets as follows:
Vitamin C (Calcium ascorbate, magnesium ascorbate): 300 mg to 1000.2 mg,
Vitamin E: 27.5 mg to 82.5 mg,
Vitamin B1: 3.3 mg to 9.9 mg,
Vitamin B2: 3.3 mg to 9.9 mg,
Vitamin B3: 115 mg to 350.1 mg,
Vitamin B5: 16.7 mg to 50.1 mg,
Vitamin B6: 3.3 mg to 9.9 mg,
Vitamin B12: 10 µg to 30 µg,
Folic acid: 133.3 µg to 399.9 µg,
Biotin: 33.3 µg to 99.9 µg,
L-carnitine: 33.3 mg to 99.9 mg,
Betaine: 23.3 mg to- 69.9 mg.

Composition mixture 1 contains selected micronutrients in a synergistic combination. This reconstructive formula can be combined with other basic formulas, e.g. Vitacor Plus^{™}.

It supplements the spectrum of specific vitamins and other micronutrients with important factors to assist the normal homocysteine and cholesterol metabolism. A tablet contains typically the following ingredients: vitamin C, cellulose filler, vitamin B3, L-carnitine tartrate 5.26%, release agent stearic acid, betaine hydrochloride 3.24%, vitamin E, vitamin B5, croscarmellose sodium, glazing agent calcium carbonate, maltodextrin, release agent silicon dioxide, glazing agent shellac, vitamin B1, vitamin B6, biotin, coloring agent riboflavin (vitamin B2), coconut oil extract, folic acid, vitamin B2, lemon oil, vitamin B12, natural lemon flavor.

Vitamins B6 and B12, folic acid and betaine are important factors for assisting the normal homocysteine metabolism. Therefore, optimal supply of these micronutrients is essential for maintaining normal homocysteine levels.

The ingredients in composition mixture 1 supports cellular metabolism in many ways simultaneously, e.g.:
a) with betaine, vitamin B6, vitamin B12 and folic acid to support normal homocysteine metabolism;
b) with biotin and B vitamins as a contribution to supporting normal energy metabolism;
c) with vitamin C and vitamin E as a contribution to protecting the cells against oxidative stress.

The recommended daily dose can be as indicated above, or can be 10 to 300%, preferably 20 to 200%, more preferably 50 to 150% thereof. The ascorbate employed is preferably obtained from identical amounts of calcium ascorbate and magnesium ascorbate.

If necessary, the composition mixture 1 and/or the statin can be combined with coenzyme Q₁₀ in a combined pharmaceutical composition or in separate pharmaceutical compositions, as outlined in US 4,929,437.

According to the present invention, composition mixture 1 is for use in treating or preventing the vascular calcification, specifically the vascular calcification induced in patients by administration of statins.

The term "treating" in this context means "mitigating" or "reversing".

The terms "mixture 1", "pharmaceutical composition mixture 1", "(pharmaceutical) composition mixture 1" or "pharmaceutical composition" are used throughout the application to represent mixture 1.

Specifically, the calcification of vascular smooth muscle cells (SMC), more specifically human aortic smooth muscle cells (AoSMC) shall be prevented or mitigated. The present invention is specifically based on the positive effect of vitamin C on vascular SMC, human dermal fibroblasts (DF) as well as immortalized human fetal osteoblasts (FOB). The process of vascular calcification requires a phenotypic transformation of vascular smooth muscle cells (VSMC) into osteogenic cells.

The concomitant administration of at least one statin and composition mixture 1 to a patient is helpful for treating or preventing cardiovascular disease, for example coronary artery disease, cerebrovascular disease or peripheral vascular disease.

By applying the present invention, the increased calcification observed under long-term statin treatments can be mitigated, reversed or prevented. Thus, there is no need for a hypothetical interpretation that the statin-induced calcification could be beneficial or that there could be a beneficial macro-calcification, as opposed to detrimental micro-calcification.

### Materials and Methods

### Reagents

Mixture 1 nutritional supplement was dissolved according to US Pharmacopea standard procedure (USP 2040 Disintegration and Dissolution of Dietary Supplements) as follows: Three recommended daily doses (nine tablets) were crushed and suspended in 900 ml 0.1 N HCl. Following one hour incubation in water bath incubator at 37°C on an orbital shaker at 75 rpm, the supplement suspension was filtered through a 0.2 mcm sterile filter, and 1 ml aliquots were frozen and stored at 20°C until use. The resulted mixture 1 solution contained 19 mM ascorbic acid according to the manufacturer's specification.

All reagents were from Sigma-Aldrich (St. Louis, MO, USA) except when indicated differently.

### Cell cultures

Normal human dermal fibroblasts (DF) and immortalized human fetal osteoblasts (hFOB) were supplied by ATCC (Manassas, VA, USA). Human aortic smooth muscle cells (AoSMC) were purchased from Cambrix (East Rutherford, NJ) and used in experiments at 5-7 passages. Cell cultures were maintained in DMEM medium (ATCC) containing antibiotics and 5% fetal bovine serum (FBS, ATCC). In some experiments cells were incubated in pro-osteogenic medium, defines as 5% FBS/DMEM fortified with 5 mM beta-glycerophosphate with or without 25 mcM forskolin. All cell cultures were maintained at 37°C and 5% CO₂ atmosphere. Cell viability was monitored with MTT assay.

### Alkaline phosphatase activity assay in AoSMC

AoSMC were plated in 96 well plates and grown to confluent layer. Cells were incubated with ascorbic acid in growth medium for three days. Cells were washed with phosphate buffered saline (PBS) and supplemented with 50 mcl/well 25 mcg/ml 4-MUP (fluorescent ALP substrate, Sigma) in alkaline buffer (Sigma)/1 % Triton X100 for 1h at room temperature. Fluorescence was measured at 360/450 nm.

### Calcium accumulation in extracellular matrix

AoSMC were seeded on fibronectin covered plastic plates at density 25,000 per square cm and grown to confluence for 5-7 days. Ascorbic acid or mixture 1 was added to cells at indicated concentrations for 72 hours in DMEM supplemented with 2% FBS and cell-produced extracellular matrix was exposed by sequential treatment with 0.5% Triton X100 and 20 mM ammonium sulfate in phosphate buffered saline (PBS, Life Technologies) for 3 min each at room temperature as described in Ivanov V, Ivanova S, Kalinovsky T, Niedzwiecki A, Rath M. Plant-derived micronutrients suppress monocyte adhesion to cultured human aortic endothelial cell layer by modulating its extracellular matrix composition. J Cardiovasc Pharmacol 2008;52:55-65. After four washes with PBS, ECM layers were solubilized by incubation in 0.6N HCl for 48 hours at 37°C. Calcium content in solubilized samples was measured with TECO Ca assay according to manufacturer's protocol.

### Expression of osteoblasts markers in human cultured cells

For the experiments AoSMC, DF and hFOB cells were seeded in separate 96 well plastic plates at density 25,000 per square cm and grown to confluence for 5-7 days. Tested compounds were added to cells at indicated concentrations for 72 hours in DMEM supplemented with 2% FBS. Cell layers were washed three times with PBS and fixed with 3% formaldehyde in PBS at 4°C for one hour. Fixed cell layers were washed four times with PBS and treated with 1% BSA/PBS for one hour at RT. Immunoassay for osteogenic markers was done by sequential incubation with primary monoclonal antibodies (R&D Systems) in 1% BSA/PBS for 2 hours followed by 1 hour incubation with secondary goat anti-mouse IgG antibodies labeled with horse radish peroxidase (HRP). Retained peroxidase activity was measured after the last washing cycle (three times with 0.1% BSA/PBS) using TMB peroxidase substrate reagent (Rockland). Optical density was read with plate reader (Molecular Devices) at 450 nm and expressed as percentage of control cell samples incubated in unsupplemented 2%FBS/DMEM. To ensure a direct comparison of osteogenic markers expression on different cell types all pcell covered plates were treated identical and simultaneously during immunoassay.

### Statistical analysis

Results in figures are means ± standard deviation SD from three or more repetitions from the most representative of at least two independent experiments. Differences between samples were estimated with a two-tailed Student's t-test using Excel software (Microsoft) and accepted as significant at p levels less than 0.05.

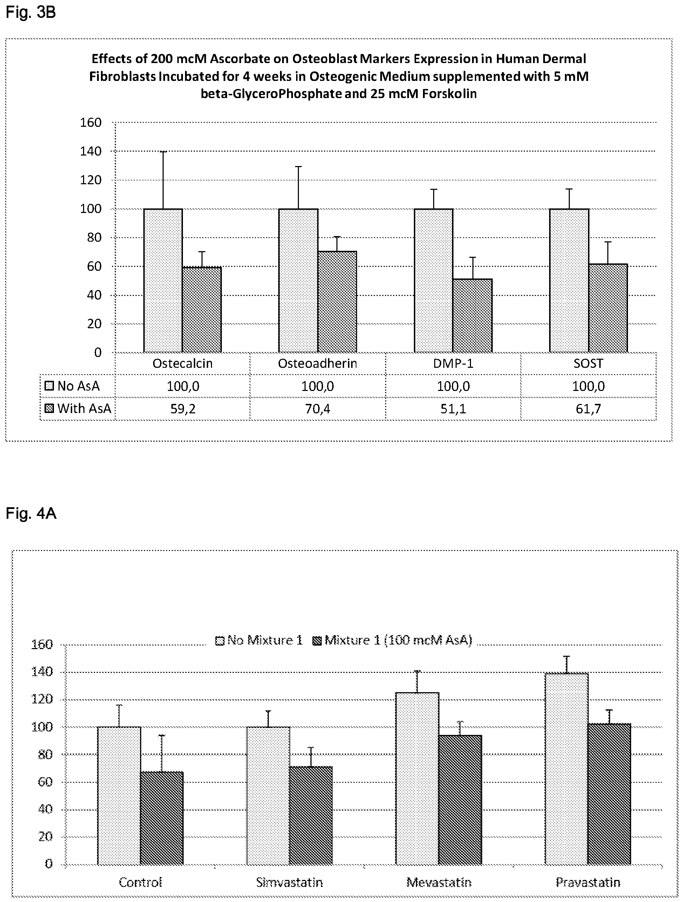

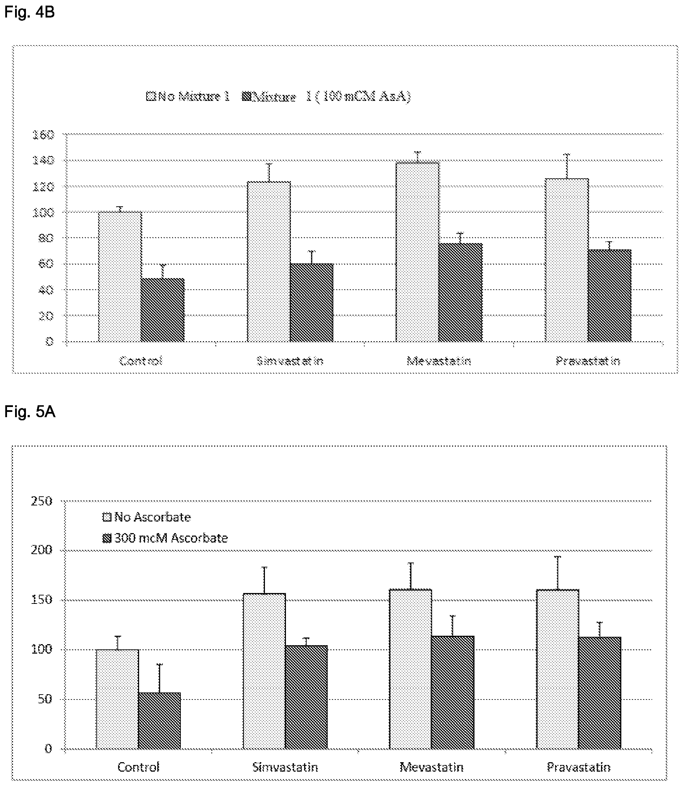

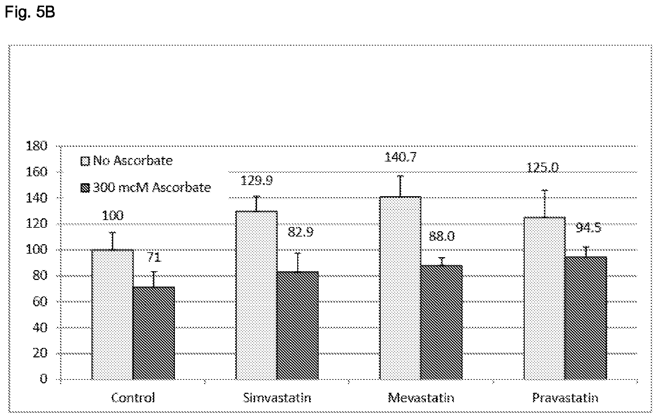

The results in Figures 4A, 4B, 5A and 5B are expressed as percentage of supplemented controls and presented as means ± SD from six or more repetitions from the most representative of at least two independent experiments.

### Results

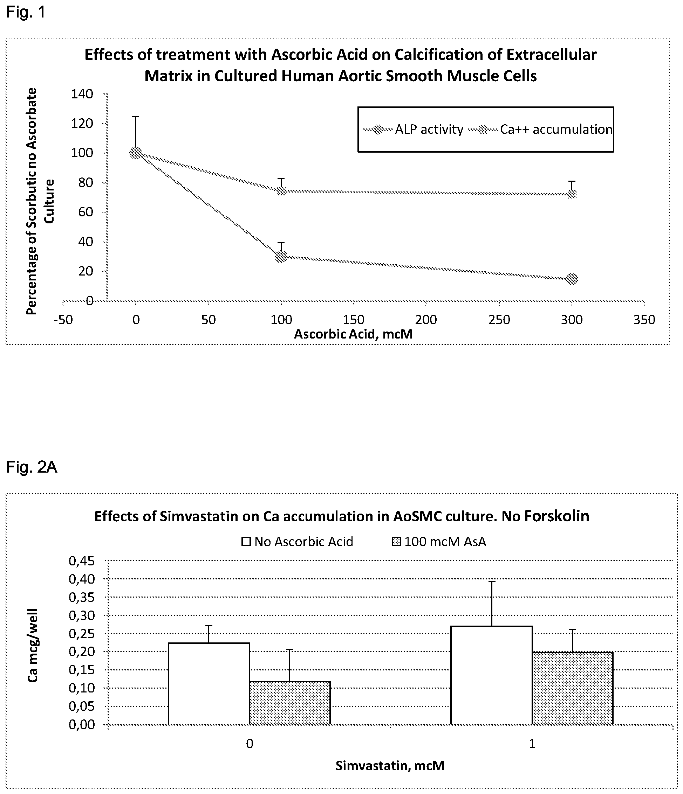

Example embodiments are illustrated by way of example and not limitation in the figures of the accompanying drawings, in which like references indicate similar elements and in which Figure 4A and Figure 4B are according to the present invention. Figure 1 shows the effects of treatment with ascorbic acid on calcification of extracellular matrix in cultured human aortic smooth muscle cells.

Figure 2A shows the effects of Simvastatin on Ca accumulation in AoSMC culture without forskolin.

Figure 2B shows the effects of mevastatin on Ca accumulation in AoSMC culture with 25 mcM forskolin.

Figure 3A shows the effects of 200 mcM ascorbate on osteoblast markers expression in human aortic SMC incubated for 4 weeks in osteogenic medium supplemented with 5 mM beta-glycerophosphate and 25 mcM forskolin.

Figure 3B shows effects of 200 mcM ascorbate on osteoblast markers expression in human dermal fibroblasts incubated for 4 weeks in osteogenic medium supplemented with 5 mM beta-glycerophosphate and 25 mcM forskolin.

Figure 4A shows effects of 1 mcM statins and Mixture 1 (at 100 mcM ascorbate) on alkaline phosphatase activity in AoSMC supplemented in plain 5% FBS/DMEM for four days. 90 min incubation with MSU substrate.

Figure 4B shows the effects of 1 mcM statins and Mixture 1 (and 100 mcM ascorbate) on alkaline phosphatase activity in AoSMC supplemented in 5 mM b-GP and 25 mcM forskolin for four days. 95 min incubation with MSU substrate.

Figure 5A shows the effects of 1 mcM statins and 300 mcM ascorbate on alkaline phosphatase activity in AoSMC supplemented in plain 5% FBS/DMEM for five days.

Figure 5B shows the effects of 1 mcM statins and 300 mcM ascorbate on alkaline phosphatase activity in AoSMC supplemented in 5% FBS/DMEM/5mM b-GP, 25 mcM forskolin for five days.

Cellular calcification process was investigated in human AoSMC cultured in a regular cell growth medium (5% FBS/DMEM) in the absence and presence of various amounts of ascorbic acid. The calcification process of AoSMC was evaluated by the activity of cellular alkaline phosphatase and calcium accumulation in the cell-produced extracellular matrix (Figure 1).

The results show that supplementation of AoSMC medium with ascorbic acid up to 300 mcM resulted in a significant decrease in the level of extracellular calcium and lower activity of cellular alkaline phosphatase in dose-dependent manner. In the presence of 300 mcM ascorbate the extracellular Ca accumulation by AoSMC decreased by 20% and alkaline phosphatase activity by 80%.

The results presented in Figure 2A show that calcium accumulation in AoSMC layers was increased in the presence of simvastatin by 23%. However, concomitant presence of 100 mcM ascorbate calcium resulted in a 54% decrease of accumulated calcium to the value 0.2 mcg/well, which correlated with the values observed in cells not exposed to simvastatin.

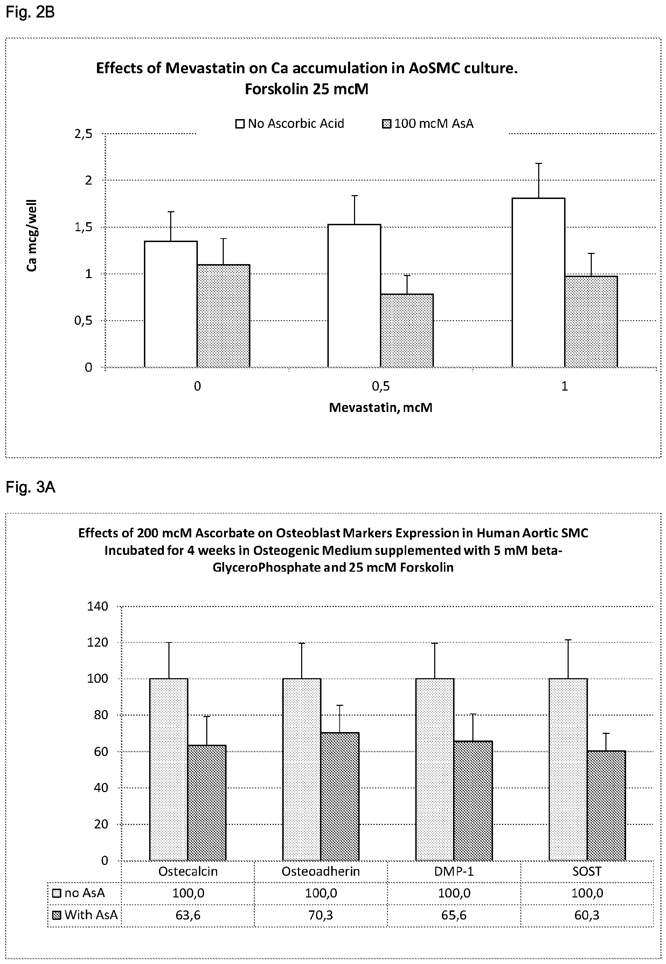

The effect of ascorbate on calcium accumulation in AoSMC under enhanced pro-calcification condition (with forskolin) and in the presence of a statin (mevastatin) is presented in Figure 2B. The results show that in the presence of 1 mM mevastatin calcium accumulation increased from 1.35 mcg/well in control to 1.8 mcg/well with mevastatin. However, when 100 mcM ascorbate was added calcium accumulation decreased by 19% to below control (non-supplemented) values.

In addition to SMC we studied the effect of ascorbate on cellular calcification process in human dermal fibroblasts (DF) and immortalized human fetal osteoblasts (FOB) by evaluating changes in the expression of different pro-osteogenic markers in these cells. The effects of ascorbate in different types of cells challenged with pro-osteogenic conditions such as by growing them in the medium supplemented with 5 mM beta-glycerophosphate and 25 mcM forskolin. The results show that expression of all tested osteogenic markers was significantly reduced by 100 mcM ascorbic acid supplementation in both AoSMC and DF cultures (Figure 3). Ascorbic acid supplementation of hFOS osteoblasts in pro-osteogenic medium over four week period was cytotoxic. Corresponding data were omitted from the presentation.

We compared the levels of osteogenic markers expression in the test human cell types as presented in Table 1. The results indicate that in a regular growth medium, the expression of osteocalcin, osteoadherin, dentin matrix protein 1 (DMP-1) and sclerostin (SOST) were most prominent in osteoblasts cells (FOB) closely followed by fibroblasts (DF), except of DMP-1, expression of which in fibroblasts slightly overcame that of FOB cultures. Cellular expression of these four osteogenic markers in AoSMC cultured in regular growth medium was significantly (2-4) fold less prominent than in FOB and DF cultures.

In the present tests we demonstrated that ascorbic acid tested up to 300 mcM concentrations can reduce calcium accumulation in ECM produced by AoSMC. This effect was accompanied by the blockage of SMC osteogenic transformation as indicated by changes in specific metabolic parameters, such as reduction in cellular alkaline phosphatase activity, and cellular expression of osteoblast marker proteins. A high level of serum alkaline phosphatase (ALP) is associated with an increased risk of mortality and myocardial infarction. ALP hydrolyses inorganic pyrophosphate, which is a strong inhibitor of calcium phosphate deposition.

**Table 1.**

| Cell type | | Osteogenic Marker | | | | | | | |
|---|---|---|---|---|---|---|---|---|---|
| | | Osteocalcin | | Osteoadherin/ OSAD | | DMP-1 | | SOST/ Sclerostin | |
| | | mean | sd | mean | sd | mean | sd | mean | sd |
| AoSMC | Plain Medium | 0,288 | 0,047 | 0,259 | 0,025 | 0,412 | 0,063 | 0,212 | 0,030 |
| | Osteogenic Medium | 0,429 | 0,086 | 0,315 | 0,061 | 0,569 | 0,111 | 0,289 | 0,063 |
| hDF | Plain Medium | 1,087 | 0,051 | 0,889 | 0,093 | 1,137 | 0,089 | 0,657 | 0,058 |
| | Osteogenic Medium | 0,614 | 0,242 | 0,403 | 0,119 | 0,851 | 0,116 | 0,374 | 0,051 |
| FOS | Plain Medium | 1,206 | 0,288 | 1,493 | 0,147 | 0,819 | 0,307 | 0,956 | 0,197 |
| | Osteogenic Medium | 1,003 | 0,207 | 1,049 | 0,213 | 0,786 | 0,078 | 0,633 | 0,126 |

Under physiological conditions (cells incubated in regular cell culture medium) expression of osteocalcin, osteoadherin and SOST/sclerostin were the highest in hFOS cultures and the lowest in hAoSMC cultures. Expression of these markers were intermediate in hDF cultures. Under physiological conditions (cells incubated in regular cell culture medium) expression of DMP-1 was the highest in hDF cultures and the lowest in hAoSMC cultures. Expression of DMP-1 was intermediate in hFOS cultures. Cell supplementation with pro-osteogenic medium as compared to regular medium caused stimulation of all tested osteomarkers in AoSMC cultures. In contrast, pro-osteogenic medium supplementation caused an inhibition of all tested osteogenic markers in hDF and hFOS cultures.

Description of the results presented on Figures 4 (A and B) and Figures 5 (A and B) shows cellular calcification process was investigated in human aortic smooth muscle cells (AoSMC) cultured in cell culture medium in the absence and presence of various statins: simvastatin, mevastatin and pravastatin used at 1 mcM concentration each. The calcification process of AoSMC was evaluated by the activity of cellular alkaline phosphatase (ALP). The study assessed the effects of mixture 1 (micronutrient combination containing ascorbate) and ascorbate individually under standard (5% FBS/DMEM) and pro-calcification conditions (5% FBS/DMEM supplemented with 5 mM beta-glycerophosphate (b-GP) and 25 mcM forskolin - Figure 4B).

The results presented on Figure 4A show that ALP activity of AoSMC under normal cell culture condition was not affected by simvastatin but it increased in the presence of mevastatin by 25%, and pravastatin by 39% compared to control. Addition of Mixture 1 at a concentration equivalent to 100 mcM ascorbate resulted in lowering of ALP activity. As such ALP was lower by 33% in control and by 29%, 25%, 27% in the presence of simvastatin, mevastatin and pravastatin, respectively. A combination of pravastatin and mixture 1 resulted in lowering calcification process to the level observed in control (unsupplemented). Mixture 1 in the presence of simvastatin and mevastatin decreased ALP activity below control values. The most effective in lowering APL activity was a combination of mixture 1 with simvastatin - 33 % below control value and almost equal to the effect of a mixture 1 applied alone.

The results presented on Figure 4B show that under pro-calcification condition (5 mM b-GP and 25 mcM forskolin) the presence of simvastatin resulted in an increase in ALP activity by 22%, with mevastatin by 39% and pravastatin by 25% compared to control. By adding mixture 1 in the presence and absence of statins, the ALP activity decreased by 52% in control and in the presence of simvastatin, mevastatin and pravastatin, by 52%, 45%, 44% respectively compared to no mixture 1 values. Similarly to normal cell culture condition, the efficacy of mixture 1 in lowering ALP activity was the highest when combined with Simvastatin (40% compared to control). Mixture 1 in combination with all test statins significantly lowered APL activity to below control levels. The significant ALP lowering effect of mixture 1 was compared to ascorbic acid used individually at 300 mcM concentration, which is three times higher than its equivalent amount contained in mixture 1 formulation.

The results on Figure 5A show that supplementation of AoSMC medium with 300 mcM ascorbic acid in the presence and absence of test statins resulted in a significant decrease in the activity of cellular alkaline phosphatase (ALP). Under normal culture medium condition, ascorbic acid decreased alkaline phosphatase activity by about 43%. In the presence simvastatin ALP activity was higher by 57% and decreased to control level after addition of 300 mcM ascorbate. In a similar way mevastatin and pravastatin increased ALP activity by 61% and 60%, respectively. These stimulatory effects were decreased by 29% and 30%, respectively, in the presence of ascorbic acid.

The results on Figure 5B show that under pro-calcification conditions statins caused an increase of 30%, 41% and 25% of the stimulation of ALP activity for simvastatin, mevastatin and pravastatin, respectively. Simultaneous supplementation with ascorbate resulted in a decrease in ALP activity by 36%, 37% and 24% for simvastatin, mevastatin and pravastatin containing samples, respectively. Under the same experimental conditions, ascorbic acid added to AoSMC in the absence of statins decreased ALP activity by 29%.

Thus, in this study we prove that mixture 1 and/or vitamin C plays a decisive role in regulating the cellular and extracellular architecture and function inside the vascular wall. With optimum availability of ascorbate or mixture 1, the integrity and stability of the vascular wall would be provided, above all, by an optimum synthesis of collagen and other ECM molecules.

## Claims

1. A composition mixture comprising L-ascorbic acid or ascorbate, vitamin E, vitamin B1, vitamin B2, vitamin B3, vitamin B5, vitamin B6, vitamin B12, folic acid, biotin, L-carnitine and betaine for use in treating or preventing the vascular calcification induced in patients by administration of statins.

2. The composition mixture according to claim 1 for use in treating patients concomitantly receiving statin treatment.

3. The composition mixture for use according to claim 1 or 2, wherein the composition mixture contains the following amounts:
L-ascorbic acid or ascorbate: 300 mg to 1000.2 mg,
vitamin E: 27.5 mg to 82.5 mg,
vitamin B1: 3.3 mg to 9.9 mg,
vitamin B2: 3.3 mg to 9.9 mg,
vitamin B3: 115 mg to 350.1 mg,
vitamin B5: 16.7 mg to 50.1 mg,
vitamin B6: 3.3 mg to 9.9 mg,
vitamin B12: 10 µg to 30 µg,
folic acid: 133.3 µg to 399.9 µg,
biotin: 33.3 µg to 99.9 µg,
L-carnitine: 33.3 mg to 99.9 mg,
betaine: 23.3 mg to 69.9 mg.

4. The composition mixture for use according to one of claims 1 to 3, wherein the ascorbate is selected from water soluble or lipid soluble ascorbates or mixtures thereof, preferably from calcium ascorbate, magnesium ascorbate, sodium ascorbate, ascorbyl phosphate, ascorbyl palmitate or mixtures thereof.

5. A pharmaceutical composition containing at least one statin and L-ascorbic acid or ascorbate, vitamin E, vitamin B1, vitamin B2, vitamin B3, vitamin B5, vitamin B6, vitamin B12, folic acid, biotin, L-carnitine and betaine in a dosage form that allows for the concomitant administering of the at least one statin and L-ascorbic acid or ascorbate and vitamin E, vitamin B1, vitamin B2, vitamin B3, vitamin B5, vitamin B6, vitamin B12, folic acid, biotin, L-carnitine and betaine to a patient.

6. The pharmaceutical composition according to claim 5 or 6, wherein the at least one statin and L-ascorbic acid or ascorbate, vitamin E, vitamin B1, vitamin B2, vitamin B3, vitamin B5, vitamin B6, vitamin B12, folic acid, biotin, L-carnitine and betaine are present as a physical mixture or as separate pharmaceutical compositions intended for concomitant administration to a patient.

7. The pharmaceutical composition according to claim 5 for use in the prevention or treatment of cardiovascular disease.

8. The pharmaceutical composition for use according to claim 7, wherein the cardiovascular disease is coronary artery disease, cerebrovascular disease or peripheral vascular disease.

9. The pharmaceutical composition according to one of claims 5 to 8, wherein the statin is selected from the group consisting of atorvastatin, cerivastatin, fluvastatin, lovastatin, mevastatin, pitavastatin, pravastatin, rosuvastatin, simvastatin, or mixtures thereof.

10. The pharmaceutical composition according to one of claims 5 to 9, wherein the ascorbate is selected from water soluble or lipid soluble ascorbates or mixtures thereof, preferably from calcium ascorbate, magnesium ascorbate, sodium ascorbate, ascorbyl phosphate, ascorbyl palmitate or mixtures thereof.

11. The pharmaceutical composition according to one of claims 5 to 10, additionally containing coenzyme Q₁₀ in a dosage form that allows for the concomitant administering of the at least one statin, L-ascorbic acid or ascorbate, and coenzyme Q₁₀ to a patient.

12. The pharmaceutical composition according to one of claims 5 to 11, comprising a daily dosage amount of L-ascorbic acid or ascorbate from 10 mg to 100 g, and of at least one statin from 5 mg to 100 mg.

13. The pharmaceutical composition according to one of claims 5 to 12, containing the following amounts:
L-ascorbic acid or ascorbate: 300 mg to 1000.2 mg,
vitamin E: 27.5 mg to 82.5 mg,
vitamin B1: 3.3 mg to 9.9 mg,
vitamin B2: 3.3 mg to 9.9 mg,
vitamin B3: 115 mg to 350.1 mg,
vitamin B5: 16.7 mg to 50.1 mg,
vitamin B6: 3.3 mg to 9.9 mg,
vitamin B12: 10 µg to 30 µg,
folic acid: 133.3 µg to 399.9 µg,
biotin: 33.3 µg to 99.9 µg,
L-carnitine: 33.3 mg to 99.9 mg,
betaine: 23.3 mg to 69.9 mg
in the pharmaceutical composition.

14. The pharmaceutical composition according to claim 13, further containing niacin, preferably in a mixture with the statin.

15. A composition mixture 1 comprising or consisting of vitamin C, vitamin E, vitamin B1, vitamin B2, vitamin B3, vitamin B5, vitamin B6, vitamin B12, folic acid, biotin, L-carnitine and betaine containing or consisting of the following amounts:
vitamin C: 300 mg to 1000.2 mg,
vitamin E: 27.5 mg to 82.5 mg,
vitamin B1: 3.3 mg to 9.9 mg,
vitamin B2: 3.3 mg to 9.9 mg,
vitamin B3: 115 mg to 350.1 mg,
vitamin B5: 16.7 mg to 50.1 mg,
vitamin B6: 3.3 mg to 9.9 mg,
vitamin B12: 10 µg to 30 µg,
folic acid: 133.3 µg to 399.9 µg,
biotin: 33.3 µg to 99.9 µg,
L-carnitine: 33.3 mg to 99.9 mg,
betaine: 23.3 mg to 69.9 mg
in the composition mixture.

16. The composition mixture 1 according to claim 15, wherein the vitamin C is in ascorbate form, wherein the ascorbate is preferably selected from water soluble or lipid soluble ascorbates, or mixtures thereof, more preferably wherein the ascorbate is selected from a calcium ascorbate, magnesium ascorbate, sodium ascorbate, ascorbyl phosphate, ascorbyl palmitate, or mixtures thereof.

17. The composition mixture 1 according to one of claims 15 or 16, additionally containing a coenzyme Q₁₀.

## Patentansprüche

1. Ein Zusammensetzungsgemisch, umfassend L-Ascorbinsäure oder Ascorbat, Vitamin E, Vitamin B1, Vitamin B2, Vitamin B3, Vitamin B5, Vitamin B6, Vitamin B12, Folsäure, Biotin, L-Carnitin und Betain zur Verwendung in der Behandlung oder Vorbeugung vaskulärer Verkalkungen, die in Patienten durch Verabreichung von Statinen induziert sind.

2. Das Zusammensetzungsgemisch nach Anspruch 1 zur Verwendung in der Behandlung von Patienten, die gleichzeitig eine Statinbehandlung erfahren.

3. Das Zusammensetzungsgemisch zur Verwendung nach Anspruch 1 oder 2, wobei das Zusammensetzungsgemisch die folgenden Mengen enthält:
L-Ascorbinsäure oder Ascorbat: 300 mg bis 1000.2 mg
Vitamin E: 27,5 mg bis 82,5 mg,
Vitamin B1: 3.3 mg bis 9.9 mg,
Vitamin B2: 3,3 mg bis 9,9 mg,
Vitamin B3: 115 mg bis 350,1 mg,
Vitamin B5: 16,7 mg bis 50,1 mg,
Vitamin B6: 3,3 mg bis 9,9 mg,
Vitamin B12: 10 µg bis 30 µg,
Folsäure: 133,3 µg bis 399,9 µg,
Biotin: 33,3 µg bis 99,9 µg,
L-Carnitin: 33,3 mg bis 99,9 mg,
Betain: 23,3 mg bis 69,9 mg.

4. Das Zusammensetzungsgemisch zur Verwendung nach einem der Ansprüche 1 bis 3, wobei das Ascorbat ausgewählt ist aus wasserlöslichen oder lipidlöslichen Ascorbaten oder deren Gemischen, vorzugsweise aus Calciumascorbat, Magnesiumascorbat, Natriumascorbat, Ascorbylphosphat, Ascorbylpalmitat oder Gemischen davon.

5. Eine pharmazeutische Zusammensetzung, enthaltend mindestens ein Statin und L-Ascorbinsäure oder Ascorbat, Vitamin E, Vitamin B1, Vitamin B2, Vitamin B3, Vitamin B5, Vitamin B6, Vitamin B12, Folsäure, Biotin, L-Carnitin und Betain in einer Dosierungsform, die die gleichzeitige Verabreichung des mindestens eines Statins mit L-Ascorbinsäure oder Ascorbat und Vitamin E, Vitamin B1, Vitamin B2, Vitamin B3, Vitamin B5, Vitamin B6, Vitamin B12, Folsäure, Biotin, L-Carnitin und Betain an einen Patienten erlaubt.

6. Die pharmazeutische Zusammensetzung nach Anspruch 5 oder 6, wobei das mindestens eine Statin und L-Ascorbinsäure oder Ascorbat, Vitamin E, Vitamin B1, Vitamin B2, Vitamin B3, Vitamin B5, Vitamin B6, Vitamin B12, Folsäure, Biotin, L-Carnitin und Betain als physikalisches Gemisch oder als separate pharmazeutische Zusammensetzungen für die gleichzeitige Verabreichung an einen Patienten vorliegen.

7. Die pharmazeutische Zusammensetzung nach Anspruch 5 zur Verwendung in der Behandlung oder Vorbeugung von Herz-Kreislauf-Erkrankungen.

8. Die pharmazeutische Zusammensetzung zur Verwendung nach Anspruch 7, wobei die Herz-Kreislauf-Erkrankungen koronare Herzkrankheit, cerebrovaskuläre Krankheit oder periphere arteriosklerotische Gefäßerkrankung sind.

9. Die pharmazeutische Zusammensetzung nach einem der Ansprüche 5 bis 8, wobei das Statin ausgewählt ist aus der Gruppe bestehend aus Atorvastatin, Cerivastatin, Fluvastatin, Lovastatin, Mevastatin, Pitavastatin, Pravastatin, Rosuvastatin, Simvastatin oder Gemischen davon.

10. Die pharmazeutische Zusammensetzung nach einem der Ansprüche 5 bis 9, wobei das Ascorbat ausgewählt ist aus wasserlöslichen oder lipidlöslichen Ascorbaten oder Gemischen davon, vorzugsweise aus Calciumascorbat, Magnesiumascorbat, Natriumascorbat, Ascorbylphosphat, Ascorbylpalmitat oder Gemischen davon.

11. Die pharmazeutische Zusammensetzung nach einem der Ansprüche 5 bis 10, zusätzlich enthaltend Coenzym Q₁₀ in einer Dosierform, die die gleichzeitige Verabreichung mit dem mindestens einen Statin, L-Ascorbinsäure oder Ascorbat und Coenzym Q₁₀ an einen Patienten erlaubt.

12. Die pharmazeutische Zusammensetzung nach einem der Ansprüche 5 bis 11, umfassend eine tägliche Dosismenge an L-Ascorbinsäure oder Ascorbat von 10 mg bis 100 g, und von mindestens einem Statin von 5 mg bis 100 mg.

13. Die pharmazeutische Zusammensetzung nach einem der Ansprüche 5 bis 12, enthaltend die folgenden Mengen
Vitamin E: 27,5 mg bis 82,5 mg,
Vitamin B1: 3.3 mg bis 9.9 mg,
Vitamin B2: 3,3 mg bis 9,9 mg,
Vitamin B3: 115 mg bis 350,1 mg,
Vitamin B5: 16,7 mg bis 50,1 mg,
Vitamin B6: 3,3 mg bis 9,9 mg,
Vitamin B12: 10 µg bis 30 µg,
Folsäure: 133,3 µg bis 399,9 µg,
Biotin: 33,3 µg bis 99,9 µg,
L-Carnitin: 33,3 mg bis 99,9 mg,
Betain: 23,3 mg bis 69,9 mg
in der pharmazeutischen Zusammensetzung.

14. Die pharmazeutische Zusammensetzung nach Anspruch 13, ferner enthaltend Niacin, vorzugsweise in einem Gemisch mit dem Statin.

15. Ein Zusammensetzungsgemisch 1 umfassend oder bestehend aus Vitamin C, Vitamin E, Vitamin B1, Vitamin B2, Vitamin B3, Vitamin B5, Vitamin B6, Vitamin B12, Folsäure, Biotin, L-Carnitin und Betain enthaltend oder bestehend aus folgenden Mengen:
Vitamin C: 300 mg bis 1000.2 mg
Vitamin E: 27,5 mg bis 82,5 mg,
Vitamin B1: 3.3 mg bis 9.9 mg,
Vitamin B2: 3,3 mg bis 9,9 mg,
Vitamin B3: 115 mg bis 350,1 mg,
Vitamin B5: 16,7 mg bis 50,1 mg,
Vitamin B6: 3,3 mg bis 9,9 mg,
Vitamin B12: 10 µg bis 30 µg,
Folsäure: 133,3 µg bis 399,9 µg,
Biotin: 33,3 µg bis 99,9 µg,
L-Carnitin: 33,3 mg bis 99,9 mg,
Betain: 23,3 mg bis 69,9 mg
im Zusammensetzungsgemisch.

16. Das Zusammensetzungsgemisch 1 nach Anspruch 15, wobei das Vitamin C in Ascorbatform vorliegt, wobei das Ascorbat vorzugsweise ausgewählt ist aus wasserlöslichen oder lipidlöslichen Ascorbaten oder Gemischen davon, stärker bevorzugt, wobei das Ascorbat ausgewählt ist aus Calciumascorbat, Magnesiumascorbat, Natriumascorbat, Ascorbylphosphat, Ascorbylpalmitat oder Gemischen davon.

17. Das Zusammensetzungsgemisch 1 nach einem der Ansprüche 15 oder 16, zusätzlich umfassend ein Coenzym Q₁₀.

## Revendications

1. Composition en mélange comprenant de l'acide L-ascorbique ou de l'ascorbate, de la vitamine E, de la vitamine B1, de la vitamine B2, de la vitamine B3, de la vitamine B5, de la vitamine B6, de la vitamine B12, de l'acide folique, de la biotine, de la L-carnitine et de la bétaïne, pour une utilisation destinée au traitement ou à la prévention de la calcification vasculaire induite chez des patients par l'administration de statines.

2. Composition en mélange selon la revendication 1, pour une utilisation destinée au traitement de patients recevant, de manière concomitante, un traitement par des statines.

3. Composition en mélange pour une utilisation selon la revendication 1 ou 2, la composition en mélange contenant les quantités suivantes :
acide L-ascorbique ou ascorbate : 300 mg à 1000,2 mg,
vitamine E : 27,5 mg à 82,5 mg,
vitamine B1 : 3,3 mg à 9,9 mg,
vitamine B2 : 3,3 mg à 9,9 mg,
vitamine B3 : 115 mg à 350,1 mg,
vitamine B5 : 16,7 mg à 50,1 mg,
vitamine B6 : 3,3 mg à 9,9 mg,
vitamine B12 : 10 µg à 30 µg,
acide folique : 133,3 µg à 399,9 µg,
biotine : 33,3 µg à 99,9 µg,
L-carnitine : 33,3 mg à 99,9 mg,
bétaïne : 23,3 mg à 69,9 mg.

4. Composition en mélange pour une utilisation selon l'une des revendications 1 à 3, dans laquelle l'ascorbate est choisi parmi les ascorbates hydrosolubles ou liposolubles, ou des mélanges de ceux-ci, préférablement parmi l'ascorbate de calcium, l'ascorbate de magnésium, l'ascorbate de sodium, le phosphate d'ascorbyle, le palmitate d'ascorbyle, ou des mélanges de ceux-ci.

5. Composition pharmaceutique contenant au moins une statine et de l'acide L-ascorbique ou de l'ascorbate, de la vitamine E, de la vitamine B1, de la vitamine B2, de la vitamine B3, de la vitamine B5, de la vitamine B6, de la vitamine B12, de l'acide folique, de la biotine, de la L-carnitine et de la bétaïne, dans une forme de dosage permettant l'administration concomitante de la au moins une statine et d'acide L-ascorbique ou d'ascorbate et de vitamine E, de vitamine B1, de vitamine B2, de vitamine B3, de vitamine B5, de vitamine B6, de vitamine B12, d'acide folique, de biotine, de L-carnitine et de bétaïne, à un patient.

6. Composition pharmaceutique selon la revendication 5 ou 6, dans laquelle la au moins une statine et l'acide L-ascorbique ou l'ascorbate, la vitamine E, la vitamine B1, la vitamine B2, la vitamine B3, la vitamine B5, la vitamine B6, la vitamine B12, l'acide folique, la biotine, la L-carnitine et la bétaïne, sont présents sous forme de mélange physique ou sous forme de compositions pharmaceutiques séparées prévus pour une administration concomitante à un patient.

7. Composition pharmaceutique selon la revendication 5, pour une utilisation destinée à la prévention ou au traitement d'une maladie cardiovasculaire.

8. Composition pharmaceutique pour une utilisation selon la revendication 7, la maladie cardiovasculaire étant une maladie coronarienne, une maladie cérébro-vasculaire ou une maladie vasculaire périphérique.

9. Composition pharmaceutique selon l'une des revendications 5 à 8, dans laquelle la statine est choisie dans le groupe constitué par l'atorvastatine, la cérivastatine, la fluvastatine, la lovastatine, la mévastatine, la pitavastatine, la pravastatine, la rosuvastatine, la simvastatine, ou des mélanges de celles-ci.

10. Composition pharmaceutique selon l'une des revendications 5 à 9, dans laquelle l'ascorbate est choisi parmi les ascorbates hydrosolubles ou liposolubles, ou des mélanges de ceux-ci, préférablement parmi l'ascorbate de calcium, l'ascorbate de magnésium, l'ascorbate de sodium, le phosphate d'ascorbyle, le palmitate d'ascorbyle, ou des mélanges de ceux-ci.

11. Composition pharmaceutique selon l'une des revendications 5 à 10, contenant en outre de la coenzyme Q₁₀ dans une forme de dosage permettant l'administration concomitante de la au moins une statine, d'acide L-ascorbique ou d'ascorbate, et de coenzyme Q₁₀, à un patient.

12. Composition pharmaceutique selon l'une des revendications 5 à 11, comprenant une quantité de dosage quotidienne d'acide L-ascorbique ou d'ascorbate allant de 10 mg à 100 g, et d'au moins une statine allant de 5 mg à 100 mg.

13. Composition pharmaceutique selon l'une des revendications 5 à 12, contenant les quantités suivantes :
acide L-ascorbique ou ascorbate : 300 mg à 1000,2 mg,
vitamine E : 27,5 mg à 82,5 mg,
vitamine B1 : 3,3 mg à 9,9 mg,
vitamine B2 : 3,3 mg à 9,9 mg,
vitamine B3 : 115 mg à 350,1 mg,
vitamine B5 : 16,7 mg à 50,1 mg,
vitamine B6 : 3,3 mg à 9,9 mg,
vitamine B12 : 10 µg à 30 µg,
acide folique : 133,3 µg à 399,9 µg,
biotine : 33,3 µg à 99,9 µg,
L-carnitine : 33,3 mg à 99,9 mg,
bétaïne : 23,3 mg à 69,9 mg,
dans la composition pharmaceutique.

14. Composition pharmaceutique selon la revendication 13, contenant en outre de la niacine, préférablement en mélange avec la statine.

15. Composition en mélange 1 comprenant, ou étant constituée, de vitamine C, de vitamine E, de vitamine B1, de vitamine B2, de vitamine B3, de vitamine B5, de vitamine B6, de vitamine B12, d'acide folique, de biotine, de L-carnitine et de bétaïne, contenant, ou étant constituée des quantités suivantes :
vitamine C : 300 mg à 1000,2 mg,
vitamine E : 27,5 mg à 82,5 mg,
vitamine B1 : 3,3 mg à 9,9 mg,
vitamine B2 : 3,3 mg à 9,9 mg,
vitamine B3 : 115 mg à 350,1 mg,
vitamine B5 : 16,7 mg à 50,1 mg,
vitamine B6 : 3,3 mg à 9,9 mg,
vitamine B12 : 10 µg à 30 µg,
acide folique : 133,3 µg à 399,9 µg,
biotine : 33,3 µg à 99,9 µg,
L-carnitine : 33,3 mg à 99,9 mg,
bétaïne : 23,3 mg à 69,9 mg,
dans la composition en mélange.

16. Composition en mélange 1 selon la revendication 15, dans laquelle la vitamine C se trouve sous forme d'ascorbate, où l'ascorbate est choisi de préférence parmi les ascorbates hydrosolubles ou liposolubles, ou des mélanges de ceux-ci, plus préférablement où l'ascorbate est choisi parmi l'ascorbate de calcium, l'ascorbate de magnésium, l'ascorbate de sodium, le phosphate d'ascorbyle, le palmitate d'ascorbyle, ou des mélanges de ceux-ci.

17. Composition en mélange 1 selon l'une des revendications 15 ou 16, contenant en outre une coenzyme Q₁₀.
